# EUROPEAN PATENT APPLICATION

(11) **EP 4 814 019 A1**
(43) Date of publication of application: **30.09.2026**
(21) Application number: 24896697.0
(22) Date of filing: 29.11.2024
(51) Int. Cl.: C07K 16/30, C12N 15/13, A61K 39/395, A61P 35/00, G01N 33/574

(54) **ANTI-NY-ESO-1 NANOBODY AND USE THEREOF**

(30) Priority: 01.12.2023 CN 202311639288
(71) Applicant: Zhejiang University, Hangzhou, Zhejiang 310058 (CN)
(72) Inventor: PAN, Liqiang, Hangzhou, Zhejiang 310058 (CN); GU, Xinyu, Hangzhou, Zhejiang 310058 (CN); HUANG, Minmin, Hangzhou, Zhejiang 310058 (CN)
(74) Representative: karo IP
(86) International application number: PCT/CN2024/135549
(87) International publication number: WO 2025/113614

(57) **Abstract**

Disclosed is an anti-NY-ESO-1 nanobody and use thereof, relating to the field of immunology. The anti-NY-ESO-1 nanobody provided by the present disclosure exhibits specific recognition and binding capacity to NY-ESO-1 antigen, with a minimum affinity constant of 68.1 nM, which demonstrates that the nanobody provided by the present disclosure possesses highly specific binding activity. The nanobody is expected to be used for the treatment of a tumor expressing NY-ESO-1 or for diagnostic detection of NY-ESO-1 protein.

## Description

### TECHNICAL FIELD

The present disclosure relates to the field of immunology, and in particular to an anti-NY-ESO-1 nanobody and use thereof.

### BACKGROUND ART

NY-ESO-1 is a member of cancer-testis antigens. It was first identified by Chen et al. via immunoscreening of a cDNA library with sera from patients with esophageal squamous cell carcinoma using the SEREX technology, and acts as a shared tumor antigen capable of inducing humoral immune responses. NY-ESO-1 is highly expressed in neuroblastoma (82%), synovial sarcoma (80%), melanoma (46%), esophageal cancer (32%), lung cancer (13%), and its expression level increases with disease progression. In contrast, its expression is extremely low or even undetectable in rectal cancer, lymphoma and other malignancies. In normal tissues, expression is restricted exclusively to germ cells and placental cells, so NY-ESO-1 is generally classified as a tumor-associated antigen. However, germ cells and placental cells lack HLA class I molecules and cannot present antigenic peptides on the cell surface for recognition by specific T cells. Accordingly, complexes formed by polypeptides derived from NY-ESO-1 degradation and HLA molecules are regarded as tumor-specific antigens. Studies have demonstrated that NY-ESO-1 can induce both humoral and cellular immune responses. Serum IgG positivity is positively correlated with the production of CD8+ T cells specific for this antigen. To date, NY-ESO-1 remains the most immunogenic tumor-specific antigen discovered (Raza A, Merhi M, Inchakalody V P, et al. Unleashing the immune response to NY-ESO-1 cancer testis antigen as a potential target for cancer immunotherapy. J Transl Med, 2020, 18(1): 140.). Given that NY-ESO-1-targeted therapies exert minimal off-target toxicity, this antigen is expected to become a highly promising candidate for tumor immunotherapy. Current research mainly focuses on tumor vaccines, TCR-mimic antibodies and T cell receptor T cell therapy (TCR-T), while no anti-NY-ESO-1 nanobody drugs are currently under development.

Sera of Camelidae contain a unique type of antibody lacking light chains, known as heavy-chain antibodies (HCAbs). A heavy-chain antibody is composed of only the variable region of the heavy chain, as well as the constant regions CH2 and CH3 of the heavy chain. The variable region of the heavy chain is termed VHH, which has a relative molecular mass of 15 kD and a molecular dimension of 4 nm × 2.5 nm × 3 nm. Such antibodies were named nanobodies (Nbs) by Ablynx. Nanobodies naturally possess only three complementarity-determining regions (CDRs), which is significantly fewer than conventional antibodies. Nevertheless, the average length of the CDR3 loop of nanobodies is greater than that of the VH domain of traditional antibodies, which enlarges the contact area with target antigens to some extent (Peter B, Julia H, Friedrich K N. Nanobodies and Nanobody-Based Human Heavy Chain Antibodies As Antitumor Therapeutics. Front Immunol, 2017, 8: 1603.). Meanwhile, the VHH CDR3 loop can naturally fold into a finger-like protrusion and insert into the cavities of antigen epitopes. This feature enables nanobodies to bind to rare or unique epitopes that are inaccessible to conventional monoclonal antibodies, such as enzyme active sites (Stijlemans B, Caljon G, Natesan S, et al. High Affinity Nanobodies against the Trypanosome brucei VSG Are Potent Trypanolytic Agents that Block Endocytosis. Plos Pathogens, 2011, 7(6)). Due to their tiny molecular size, nanobodies feature efficient tissue distribution and can easily penetrate dense solid tumors to exert therapeutic effects, showing great prospects for clinical application. In addition, nanobodies also have advantages including high water solubility, excellent stability and low production cost, which enable their wide application in disease diagnosis and treatment.

### SUMMARY

In order to overcome the deficiencies of the prior art, an objective of the present disclosure is to provide an anti-NY-ESO-1 nanobody and use thereof, wherein the nanobody has strong affinity and specificity for NY-ESO-1.

In a first aspect, the present disclosure provides an anti-NY-ESO-1 nanobody, wherein a VHH chain of the nanobody comprises three complementarity determining regions CDR1, CDR2, and CDR3, an amino acid sequence of the CDR1 is any one of the sequences set forth in SEQ ID NO.1, SEQ ID NO.6, SEQ ID NO.11, and SEQ ID NO.16 ; an amino acid sequence of the CDR2 is any one of the sequences set forth in SEQ ID NO.2, SEQ ID NO.7, SEQ ID NO.12, and SEQ ID NO.17 ; and an amino acid sequence of the CDR3 is any one of the sequences set forth in SEQ ID NO.3, SEQ ID NO.8, SEQ ID NO.13, and SEQ ID NO.18.

Preferably, the VHH chain of the nanobody comprises three complementarity determining regions CDR1, CDR2, and CDR3 selected from any one of the following: (1) a CDR1 having an amino acid sequence set forth in SEQ ID NO.1, a CDR2 having an amino acid sequence set forth in SEQ ID NO.2, and a CDR3 having an amino acid sequence set forth in SEQ ID NO.3; (2) a CDR1 having an amino acid sequence set forth in SEQ ID NO.6, a CDR2 having an amino acid sequence set forth in SEQ ID NO.7, and a CDR3 having an amino acid sequence set forth in SEQ ID NO.8; (3) a CDR1 having an amino acid sequence set forth in SEQ ID NO.11, a CDR2 having an amino acid sequence set forth in SEQ ID NO.12, and a CDR3 having an amino acid sequence set forth in SEQ ID NO.13; and (4) a CDR1 having an amino acid sequence set forth in SEQ ID NO.16, a CDR2 having an amino acid sequence set forth in SEQ ID NO.17, and a CDR3 having an amino acid sequence set forth in SEQ ID NO.18. An amino acid sequence of the VHH chain is any one of the sequences set forth in SEQ ID NO.4, SEQ ID NO.9, SEQ ID NO.14, and SEQ ID NO.19.

In another preferred embodiment, the CDR1 has an amino acid sequence with at least 80% sequence identity to SEQ ID NO.1, SEQ ID NO.6, SEQ ID NO.11 or SEQ ID NO.16; and/or the CDR2 has an amino acid sequence with at least 80% sequence identity to SEQ ID NO.2, SEQ ID NO.7, SEQ ID NO.12 or SEQ ID NO.17; and/or the CDR3 has an amino acid sequence with at least 80% sequence identity to SEQ ID NO.3, SEQ ID NO.8, SEQ ID NO.13 or SEQ ID NO.18.

In a second aspect, the present disclosure provides a multivalent nanobody complex comprising at least two nanobody molecules, wherein the nanobodies are the nanobodies described above. In a third aspect, the present disclosure provides a nucleic acid molecule encoding the nanobody. The nucleic acid molecule comprises a nucleotide sequence set forth in any one of SEQ ID NO.5, SEQ ID NO.10, SEQ ID NO.15, and SEQ ID NO.20. In a fourth aspect, the present disclosure provides an expression vector comprising the nucleic acid molecule. The expression vector is pET-21a(+). In a fifth aspect, the present disclosure provides a host cell; the host cell comprises the expression vector, or the nucleic acid molecule is integrated into a genome of the host cell. The host cell is Escherichia coli BL21. In a sixth aspect, the present disclosure provides an immunoconjugate comprising: (a) the nanobody; and (b) at least one conjugation moiety selected from the group consisting of a detectable marker, a drug, toxin, a cytokine, a therapeutic agent, a PK modification moiety, and an enzyme.

Preferably, the therapeutic agent is a CAR; more preferably, the therapeutic agent is an anti-CD3 antibody.

In another preferred embodiment, the immunoconjugate comprises: a multivalent (e.g., bivalent) anti-NY-ESO-1 nanobody according to the first aspect of the present disclosure, or a multivalent nanobody complex according to the second aspect of the present disclosure. The term "multivalent" means that the amino acid sequence of the immunoconjugate contains multiple repeats of the anti-NY-ESO-1 nanobody according to the first aspect of the present disclosure or the multivalent nanobody complex according to the second aspect of the present disclosure.

In a seventh aspect, the present disclosure provides a pharmaceutical composition comprising the nanobody, the nucleic acid molecule, the immunoconjugate, and a pharmaceutically acceptable carrier. In an eighth aspect, the present disclosure provides use of the nanobody, the nucleic acid molecule, the host cell, or the immunoconjugate, comprising any one of the following: (1) use in preparation of a product for detecting NY-ESO-1; (2) use in preparation of a product that binds to NY-ESO-1; (3) use in preparation of a product for diagnosing or treating a tumor expressing NY-ESO-1. The use is for non-diagnostic and non-therapeutic purposes. The product is a pharmaceutical agent, a reagent, a detection plate, a kit, or the like. The NY-ESO-1-specific nanobody of the present disclosure can be used for treating any NY-ESO-1-related disease that presents the complex of NY-ESO-1 antigenic short peptide SLLMWITQC-HLA A0201 complex, including but not limited to tumors, preferably, the tumors include but are not limited to: neuroblastoma, sarcoma, melanoma, prostate cancer, bladder cancer, breast cancer, multiple myeloma, hepatocellular carcinoma, oral squamous cell carcinoma, esophageal cancer, gastric cancer, lung cancer, head and neck squamous cell carcinoma, colon cancer, ovarian cancer, and the like.

Beneficial effects of the present disclosure are as follows.

The anti-NY-ESO-1 nanobody provided by the present disclosure exhibits specific recognition and binding capacity to an NY-ESO-1 antigen, with a minimum affinity constant of 68.1 nM, which demonstrates that the nanobody provided by the present disclosure possesses highly specific binding activity. The nanobody is expected to be used for the treatment of a tumor expressing NY-ESO-1 or for diagnostic detection of a NY-ESO-1 protein.

### BRIEF DESCRIPTION OF THE DRAWINGS

FIG. 1 shows the SDS-PAGE gel image of the purified inclusion bodies; of which, lane 2 is HLA-A2, and lane 3 is β2m.
FIG. 2 shows the SDS-PAGE gel image of the SLLMWITQC-HLA A0201 complex purified by size-exclusion chromatography; of which, lane 1 is the polymer, lane 2 is β2m, and lanes 3-8 are SLLMWITQC-HLA A0201 complex.
FIG. 3 shows the ELISA identification results of the SLLMWITQC-HLA A0201 complex.
FIG. 4 shows the expression rate and positivity rate of nanobodies after magnetic sorting.
FIG. 5 shows the change in the proportion of double-positive cells after four rounds of flow cytometric sorting.
FIG. 6, FIG. 7, FIG. 8, and FIG. 9 show the comparison of binding capacities of VHH-1, VHH-4, VHH-5, and VHH-9 monoclonal antibodies to positive and negative antigens, respectively.
FIG. 10 shows BIAcore kinetics profiles of the binding of VHH-1, VHH-4, VHH-5 and VHH-9 to the SLLMWITQC-HLA A0201 complex.
FIG. 11 shows SDS-PAGE gel images of dimers and tetramers.
FIG. 12 shows the comparison of binding capacities of dimers to positive and negative antigens.
FIG. 13 shows the comparison of binding capacities of tetramers to positive and negative antigens.

### DETAILED DESCRIPTION OF THE EMBODIMENTS

### Definitions

Nanobodies, also known as single-domain antibodies, are a class of naturally light-chain-deficient antibodies found in the peripheral blood of alpacas. Such antibodies consist solely of one variable domain of the heavy chain (VHH) and two conventional constant domains CH2 and CH3. The individually cloned and expressed VHH domain possesses structural stability and antigen-binding activity comparable to those of the parent heavy-chain antibody. As the smallest known functional unit capable of binding to target antigens, it is thus named Nanobody (Nb).

Major histocompatibility complex (MHC) molecules belong to the immunoglobulin superfamily and are classified into MHC class I and MHC class II molecules. Therefore, they exhibit specificity in antigen presentation. Individuals carry distinct MHC molecules, which can present different short peptides derived from a single protein antigen onto the surface of their respective antigen-presenting cells (APCs). In humans, MHC molecules are generally referred to as human leukocyte antigen (HLA) genes or the HLA complex.

An anti-CD3 antibody refers to an antibody that specifically binds to a single CD3 chain (e.g., CD3(γ) chain, CD3(δ) chain or CD3(ε) chain) or a complex formed by two or more CD3 chains (e.g., a complex containing more than one CD3(ε) chain, a complex of CD3(γ) chain and CD3(ε) chain, or a complex of CD3(δ) chain and CD3(ε) chain). In certain embodiments, the anti-CD3 antibody specifically binds to CD3(γ), CD3(δ), CD3(ε), or any combination thereof. More preferably, it specifically binds to CD3(ε). Human-derived CD3 is denoted as hCD3. Accordingly, the terms "anti-human CD3 antibody" and "anti-hCD3 antibody" both refer to an antibody that specifically binds to human-derived CD3.

The "Fc" region of an antibody refers to the portion of the antibody formed by the second (CH2) and third (CH3) constant domains of the first heavy chain linked via disulfide bonds to the second and third constant domains of the second heavy chain. The Fc region mediates multiple effector functions, such as antibody-dependent cell-mediated cytotoxicity (ADCC) and complement-dependent cytotoxicity (CDC), but does not participate in antigen binding. A tumor refers to a disease characterized by pathological proliferation of cells or tissues, accompanied by subsequent migration or invasion into other tissues or organs. Tumor growth is typically uncontrolled and progressive, and does not induce or inhibit the proliferation of normal cells. The NY-ESO-1-specific nanobody of the present disclosure can be used for treating any NY-ESO-1-related disease that presents the complex of NY-ESO-1 antigenic short peptide SLLMWITQC-HLA A0201 complex. Such diseases include, but are not limited to, tumors. Preferably, the tumors include neuroblastoma, sarcoma, melanoma, prostate cancer, bladder cancer, breast cancer, multiple myeloma, hepatocellular carcinoma, oral squamous cell carcinoma, esophageal cancer, gastric cancer, lung cancer, head and neck squamous cell carcinoma, colon cancer, ovarian cancer, and the like.

### Example 1 Preparation of SLLMWITQC-HLA A0201 Complex

### 1.1 Purification of Inclusion Bodies

Bacterial cultures induced to express HLA-A2 and β2m were collected and centrifuged at 4000 rpm for 30 min. The supernatant was completely removed, and the pellet was resuspended in 40 mL re-suspension buffer (25% Sucrose, 2 M Tris, pH 8.0, 1 mM EDTA, 1 mM PMSF, 1 mM DTT). The suspension was transferred into 30 mL centrifuge tubes and stored at -80 °C.

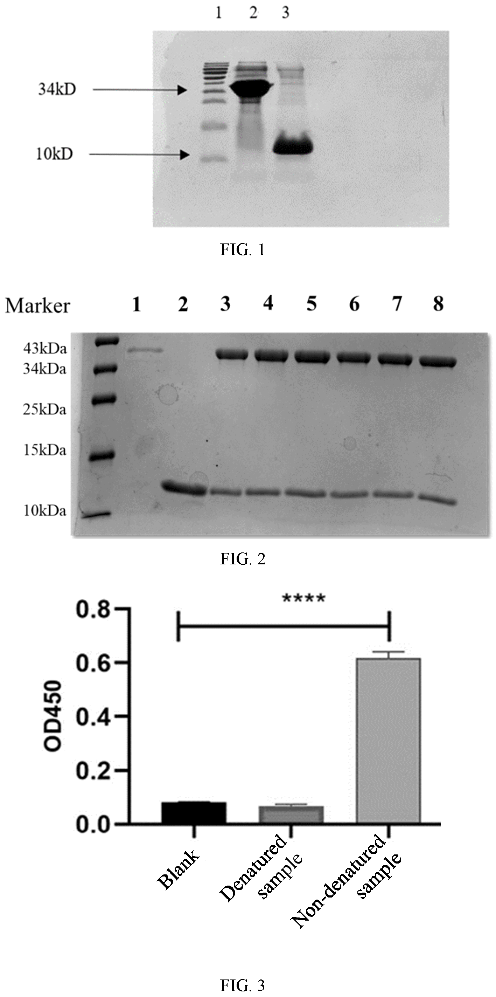

The frozen bacterial suspension was taken out from the -80 °C refrigerator and thawed at room temperature. At 4 °C, 400 µL of 1 M MgCl₂, 400 µL of 10 mg/mL DNase and 400 µL of 100 mM DTT were added sequentially. The mixture was stirred for 10 min, and cell disruption was then performed using a cell disruptor. The mixture was centrifuged at 20,000 × g for 20 min at 4 °C. The resulting pellet was resuspended and washed with 40 mL detergent buffer (0.1% NP-40, 0.2 M NaCl, 20 mM Tris, pH 7.5, 2 mM EDTA). Subsequently, the pellet was washed twice with 40 mL wash buffer I (0.5% Triton X-100, 50 mM Tris, pH 8.0, 100 mM NaCl) and twice with 40 mL wash buffer II (100 mM Tris, pH 8.0, 150 mM NaCl, 1 mM EDTA). The final pellet was resuspended in 1 mL distilled water, and 4 mL denaturing buffer (10 M Urea, 62.5 mM MES, pH 6.5, 0.125 mM EDTA, 0.125 mM DTT) was added. The centrifuge tube was inverted continuously on a rotary mixer for 30 min to achieve complete denaturation and solubilization of proteins. After centrifugation following the same procedure, the protein-containing supernatant was collected for concentration measurement, and the raw readings were corrected using extinction coefficients. The actual protein concentration (mg/mL) was calculated by the formula: Measured reading (mg/mL) / ε₂₈₀. The ε₂₈₀ values were 2.28 for HLA-A2 and 1.56 for β2m. Protein samples were analyzed by SDS-PAGE, and the results are shown in FIG. 1. The bands corresponding to HLA-A2 and β2m were consistent with the expected molecular weights, and high protein purity was achieved.

### 1.2 Refolding of pMHC

1.54 g reduced glutathione, 0.31 g oxidized glutathione and 5 mL of 100 mM PMSF were dissolved in 1 L folding buffer (400 mM L-Arginine, 100 mM Tris, pH 8.0, 2 mM EDTA). NY-ESO-1 ₁₅₇₋₁₆₅ (SLLMWITQC) peptide was dissolved in DMF to 20 mg/mL, and 0.5 mL was added to the reaction mixture. β2m was dissolved in folding buffer to prepare a 5 mL solution, which was added to the reaction mixture in three separate portions. Afterwards, HLA-A2 was dissolved in injection buffer (3 M Guanidine Hydrochloride, 10 mM Sodium Acetate, 10 mM EDTA) to prepare a 5 mL solution, which was also added into the reaction system in three portions. The refolding reaction was carried out at 4 °C for two days.

### 1.3 Biotinylation

The refolding mixture was centrifuged at 20,000 × g for 10 min at 4 °C, and the supernatant was filtered through a 0.22 µm microporous membrane. The filtrate was concentrated and buffer-exchanged with 1× PBS using a Millipore 30 kD ultrafiltration membrane at 4 °C and 4500 rpm. Buffer exchange was continued until PBS accounted for more than 80% of the total volume, and the final volume was approximately 5 mL. To the reaction system, 5 mM MgCl₂, 5 mM ATP, 100 µM Biotin and 10 µg/mL BirA were added in sequence. The mixture was incubated in a water bath at 30 °C for 1 h. The reaction solution was centrifuged at 20,000 × g for 5 min at 4 °C. The supernatant was collected and further ultrafiltered to 600 µL using a 30 kD ultrafiltration membrane.

### 1.4 Purification of Refolded Product by Size-Exclusion Chromatography

A total of 600 µL biotinylated pMHC molecules were loaded onto a Superdex 75 Increase 10/300 GL column (GE Healthcare Life Sciences) using an AKTA pure 25 L1 protein purification system. Elution was performed with PBS at a flow rate of 0.75 mL/min. The biotinylated pMHC molecules were eluted at an elution volume of around 10 mL. The collected fractions were identified by SDS-PAGE, and the results are presented in FIG. 2. The bands of refolded pMHC were consistent with the expected molecular weights, and high purity was achieved.

### 1.5 Identification of pMHC by ELISA

Streptavidin was diluted to 1 µg/mL with coating buffer, and 200 µL of was added to each well. The plate was incubated overnight at 4 °C. After coating, the liquid in each well was discarded, and the wells were washed four times with water. Then 200 µL blocking buffer was added to each well, and the plate was incubated at 37 °C for 1.5 h. The liquid was discarded, and 100 µL of sample or denatured sample was added to each well. The plate was incubated at 37 °C for 1.5 h. The wells were washed four times with water, and 100 µL W6/32 antibody solution was added to each well, followed by incubation at 37 °C for 1.5 h. After washing 6 times with water, 100 µL HRP-conjugated goat anti-mouse IgG antibody was added to each well, and the plate was incubated at 37 °C for 1 h. The wells were washed nine times with water. Under dark conditions, 100 µL chromogenic substrate solution was added to each well, and the plate was incubated at 37 °C for 30 min. 100 µL of 2 M H₂SO₄ was added to each well to stop the reaction, and the absorbance was measured at 450 nm using a microplate reader. The ELISA results are shown in FIG. 3. Significant binding between non-denatured sample and W6/32 was detected compared with the blank and denatured samples, which indicated that the refolded pMHC possessed correct conformation.

### Example 2 Screening of Nanobodies

### Construction of Yeast Display Library for Nanobodies

A nanobody DNA library was constructed by two-step overlap extension PCR. Ten primers (one set) including P1_for, P2_rev ... P10_rev were dissolved to a concentration of 100 µM and mixed at an equal molar ratio to prepare three primer pools, each containing primers at a final concentration of 10 µM. The three primer pools (Short Pool, Medium Pool and Long Pool) differed in the P9 primer region. P9a_for, P9b_for and P9c_for were used to introduce complementarity determining region 3 (CDR3) with 7, 11 and 15 random residues of variable length, respectively. Subsequently, 1 µL of each 10 µM primer pool (serially diluted 5-fold) was used to set up 50 µL overlap extension PCR reactions with high-fidelity polymerase. Full-length nanobody DNA products amplified from the three pools were mixed at a molar ratio of 1:2:1 (Short CDR3: Medium CDR3: Long CDR3), representing the length distribution frequency observed in camelid VHH domains. The resulting mixture was amplified using primers pET26b_NbLib_GA_for and pET26b_NbLib_GA_rev and cloned into pET26b. Preliminary verification of these synthetic nanobodies was carried out in *Escherichia coli*.

The nanobody DNA library was amplified sequentially with primer pairs pYDSFor1/pYDSRev1, pYDSFor2/pYDSRev2 and pYDSFor3/pYDSRev2 for subsequent yeast transformation. When 500 mL of BJ5465 yeast culture was grown to an OD₆₀₀ of 1.5, the yeast cells were electrotransformed with 245 µg nanobody insert DNA and 50 µg of *Nhe*I-HF/*Bam*HI-HF double-digested pYDS649 plasmid. Diluted transformed yeast cells were spread onto tryptophan-deficient selective agar plates to form single colonies, and the diversity of the constructed library was estimated. The primers used are shown in Table 1.

**Table 1 Sequences of Primers**

| Primer name | Primer sequence |
|---|---|
| P1_for | caggtgcagctgcaggaaagcggcggcggcctggtgcaggcgggcggcag |
| P2_rev | gccgctcgccgcgcagctcaggcgcaggctgccgcccgcctgc |
| P3_for | cgcggcgagcggcWMTattTYTNNNNatgggctggtatcgccagg |
| P4_rev | ttcgcgttctttgcccggcgcctggcgataccagcccat |
| P5_for | ccgggcaaagaacgcgaaYTTgttgccRSTattRVTNggtRSTANTaccWATtatgcggatagcg tgaaaggcc |
| P6_rev | gtttttcgcgttatcgcggctaatggtaaagcggcctttcacgctatccgcata |
| P7_for | agccgcgataacgcgaaaaacaccgtgtatctgcagatgaacagcctgaaacc |
| P8_rev | cgcgcaataatacaccgcggtatcttccggtttcaggctgttcatctgcaga |
| P9a_for | ccgcggtgtattattgcgcgGYTNNNNNNNYWTNtattggggccagggcacc |
| P9b_for | ccgcggtgtattattgcgcgGYTNNNNNNNNNNNYWTNtattggggccagggcacc |
| P9c_for | ccgcggtgtattattgcgcgGYTNNNNNNNNNNNNNNNYWTNtattggggccagggcacc |
| P10_rev | gctgctcacggtcacctgggtgccctggccccaata |
| pET26b_NbLib _GA_for | CTCCTCGCTGCCCAGCCGGCGATGGCCcaggtgcagctgcaggaaag |
| pET26b_NbLib_GA_rev | CGGATCTCAGTGGTGGTGGTGGTGGTGgctgctcacggtcacctgg |
| pYDSFor1 | GAAGGTGTTCAATTGGACAAGAGAGAAGCTGACGCAcaggtgcagctgcagg aaagcggc |
| pYDSRev1 | TACTGATGCTTCTGTAGAGGGTGAGGATGTTTGAGCGTAATCTGGAAC ATCGTATGGGTAGGATCCgctgctcacggtcacctgggtgcc |
| pYDSFor2 | ACCACCATCGCTTCTATCGCTGCTAAGGAAGAAGGTGTTCAATTGGAC AAGAGAGAAGC |
| pYDSRev2 | TACTGATGCTTCTGTAGAGGGTGAGG |
| pYDSFor3 | ACCACCATCGCTTCTATCGCTGCTAAG |

### 2.1 Inducible Expression of Yeast Display Nanobody Library

The yeast display nanobody library was taken out from the -80 °C freezer and thawed in a 30 °C thermostatted water bath. The cell suspension was centrifuged at 2000 × g for 5 min at 4 °C. The supernatant was discarded, and the cell pellet was resuspended in a small volume of growth medium. The resuspended yeast cells were added to 1 L growth medium and cultured overnight at 30 °C with shaking at 220 rpm until the OD₆₀₀ value reached 2-3. Subsequently, the culture was centrifuged at 2000 × g for 5 min at 4 °C, and the supernatant was removed. The cell pellet was resuspended in induction medium, and the OD₆₀₀ was adjusted to 1. The culture was incubated at 25 °C with shaking at 220 rpm for 18-24 h. Finally, the initial nanobody expression level (ER1) was evaluated by flow cytometry.

### 2.2 First Round of Magnetic-Activated Cell Sorting (MACS)

Magnetic beads (Invitrogen, 11205D) were washed twice with 1 mL selection buffer. After the supernatant was aspirated, the beads were resuspended in 400 µL selection buffer. A yeast culture equivalent to 2000 OD₆₀₀ units was centrifuged at 2000 × g for 5 min at 4 °C. The supernatant was discarded, and the cells were resuspended in 20 mL selection buffer followed by a second centrifugation. The supernatant was discarded and the cells were resuspended in 10 mL selection buffer. A total of 200 µL magnetic bead suspension was added to the yeast suspension and mixed thoroughly. The mixture was incubated on a rotary mixer with gentle rotation for 30 min at 4 °C. The mixture was divided into 1.5 mL centrifuge tubes (1 mL per tube) and placed on a magnetic stand for 2-3 min. Unbound yeast cells were aspirated and collected into a 30 mL centrifuge tube. The magnetic beads were washed with selection buffer and combined into the same tube. The combined cell suspension was centrifuged at 2000 × g for 5 min at 4 °C. The supernatant was discarded, and the cells were resuspended in 5 mL selection buffer.

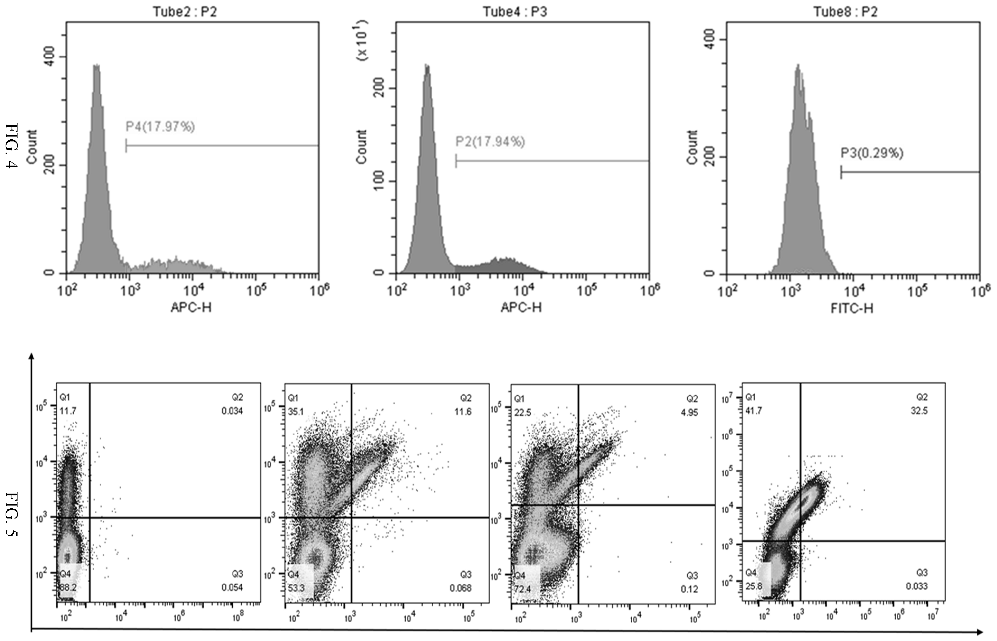

NY-ESO-1 antigen was added to a final concentration of 200 nM, and the mixture was incubated on a rotary mixer with gentle rotation for 1 h at 4 °C. The mixture was centrifuged at 2000 × g for 5 min at 4 °C. The supernatant was aspirated, and the cells were resuspended in 10 mL selection buffer and centrifuged again. The final pellet was resuspended in 5 mL selection buffer. 200 µL magnetic bead suspension was added and mixed well, and the mixture was incubated on a rotary mixer for 30 min at 4 °C. The mixture was transferred to centrifuge tubes and placed on a magnetic stand for 2-3 min. Unbound yeast cells were removed, and the magnetic beads were washed twice with 1 mL selection buffer per tube. The beads carrying bound yeast cells were resuspended in growth medium and transferred into a 50 mL conical flask. Additional growth medium was supplemented until the total volume reached 15 mL. The culture was incubated overnight at 30 °C with shaking at 220 rpm until the OD₆₀₀ reached 2-3. Yeast cells equivalent to 50 OD₆₀₀ units were centrifuged at 2000 × g for 5 min at 4 °C. The medium was discarded, and the cells were resuspended in 20 mL induction medium and centrifuged again. The upper medium was aspirated, and the cells were resuspended in induction medium with the OD₆₀₀ adjusted to 1. The cells were cultured overnight at 25 °C with shaking at 220 rpm. The nanobody expression level (ER2) and positive cell rate after the first round of MACS were detected by flow cytometry, and the results are shown in FIG. 4. The initial HA expression rate of the yeast library was 17.97%. No obvious enrichment of nanobody-expressing cells was observed after one round of magnetic sorting, and the proportion of cells bound to NY-ESO-1 antigen was 0.29%. Although the sorting result was not as expected, the total library capacity was reduced by 2 to 3 orders of magnitude after MACS.

### 2.3 Second Round of MACS

The dosage of yeast culture was adjusted to 100 OD₆₀₀ units, the volume of magnetic beads was changed to 80 µL, and the antigen concentration was set to 100 nM. All other experimental operations were performed in accordance with the procedures described in Section 2.2.

### 2.4 First Round of Fluorescence-Activated Cell Sorting (FACS)

Yeast cells from the second round of MACS were stained with APC-labeled anti-HA antibody (Invitrogen, 26183-A647) and FITC-labeled streptavidin protein (Abcam, ab136201). The nanobody expression level and positive cell rate were analyzed by a flow sorter. APC and FITC double-positive cells were sorted and collected into flow tubes containing 1.5 mL growth medium. The sorted target yeast cells were spread onto solid growth medium and cultured statically at 30 °C for 1-2 days. After abundant colonies were formed, yeast cells on the solid medium were washed off with growth medium. The cell suspension was centrifuged at 2000 × g for 5 min at 4 °C. The growth medium supernatant was aspirated, and the yeast cells were resuspended in 5 mL induction medium and centrifuged again. The cells were diluted with induction medium to an OD₆₀₀ of 1 and cultured overnight at 25 °C with shaking at 220 rpm.

### 2.5 Second Round of FACS and Detection

TP53-R273H was used as the antigen in this round. The antigen concentration, grouping and experimental operations were the same as those described in Section 2.4.

### 2.6 Third Round of FACS and Detection

NY-ESO-1 was used as the antigen at a concentration of 50 nM. The grouping and experimental procedures were the same as those described in Section 2.4.

### 2.7 Fourth Round of FACS and Detection

The antigen, concentration, grouping and experimental procedures were the same as those described in Section 2.6.

The results of four rounds of FACS are shown in FIG. 5. After four rounds of screening, the nanobody expression rate increased from 12.7% to 74.2%, and the proportion of double-positive cells rose to 32.5%, which indicated a significant enrichment effect.

### Example 3 Identification and Analysis of Specific Yeast Clones

### 3.1 Sequence Analysis of Yeast Monoclones

A total of 5 µL overnight-cultured yeast cells from the fourth round of FACS in Example 2 were transferred into 1 mL growth medium and mixed thoroughly. 50 µL of the mixture was spread onto solid growth medium and cultured for 1-2 days. Ten single colonies were picked randomly and inoculated into 1 mL growth medium, and incubated at 30 °C with shaking at 180 rpm for 24 h. The ten yeast monoclones were sent to Tsingke Biotechnology for sequencing. Four different nanobodies were obtained by sequencing, as shown in Table 2.

**Table 2**

| Antibody No. | Type | SEQ ID No: | Sequence |
|---|---|---|---|
| NY-ESO-1-VHH-1 | CDR1 amino acid sequence | 1 | GTIFHQIDM |
| | CDR2 amino acid sequence | 2 | ELVAAIGTGSSTYY |
| | CDR3 amino acid sequence | 3 | AVYYCAAWHYY |
| | Amino acid sequence of heavy chain variable region | 4 | QVQLQESGGGLVQAGGSLRLSCAASGTIFH QIDMGWYRQAPGKERELVAAIGTGSSTYY ADSVKGRFTISRDNAKNTVYLQMNSLKPE DTAVYYCAAWHYYWGQGTQVTVSS |
| | Nucleotide sequence of heavy chain variable region | 5 | CAGGTGCAGCTGCAGGAAAGCGGCGGC GGCCTGGTGCAGGCGGGCGGCAGCCTGC GCCTGAGCTGCGCGGCGAGCGGCACTAT TTTTCATCAGATCGACATGGGCTGGTATC GCCAGGCGCCGGGCAAAGAACGCGAAC TTGTTGCCGCTATTGGTACTGGTAGTAGT ACCTATTATGCGGATAGCGTGAAAGGCCG CTTTACCATTAGCCGCGATAACGCGAAAA ACACCGTGTATCTGCAGATGAACAGCCTG AAACCGGAAGATACCGCGGTGTATTATTG CGCGGCTTGGCATTACTATTGGGGCCAGG GCACCCAGGTGACCGTGAGCAGC |
| NY-ESO-1-VHH-4 | CDR1 amino acid sequence | 6 | GNIFYPQYM |
| | CDR2 amino acid sequence | 7 | EFVAAISDGASTYY |
| | CDR3 amino acid sequence | 8 | AVYYCADLWY |
| | Amino acid sequence of heavy chain variable region | 9 | QVQLQESGGGLVQAGGSLRLSCAASGNIF YPQYMGWYRQAPGKEREFVAAISDGASTY YADSVKGRFTISRDNAKNTVYLQMNSLKP EDTAVYYCADLWYWGQGTQVTVSS |
| | Nucleotide sequence of heavy chain variable region | 10 | CAGGTGCAGCTGCAGGAAAGCGGCGGC GGCCTGGTGCAGGCGGGCGGCAGCCTGC GCCTGAGCTGCGCGGCGAGCGGCAATAT TTTTTACCCGCAGTACATGGGCTGGTATC GCCAGGCGCCGGGCAAAGAACGCGAATT TGTTGCCGCTATTAGTGACGGTGCTAGTA CCTATTATGCGGATAGCGTGAAAGGCCGC TTTACCATTAGCCGCGATAACGCGAAAAA CACCGTGTATCTGCAGATGAACAGCCTGA AACCGGAAGATACCGCGGTGTATTATTGC GCGGACCTTTGGTATTGGGGCCAGGGCA CCCAGGTGACCGTGAGCAGC |
| NY-ESO-1-VHH-5 | CDR1 amino acid sequence | 11 | GNISAVYTM |
| | CDR2 amino acid sequence | 12 | EFVAAIDYGGSTYY |
| | CDR3 amino acid sequence | 13 | AVYYCASYYHGY |
| | Amino acid sequence of heavy chain variable region | 14 | QVQLQESGGGLVQAGGSLRLSCAASGNISA VYTMGWYRQAPGKEREFVAAIDYGGSTY YADSVKGRFTISRDNAKNTVYLQMNSLKP EDTAVYYCASYYHGYWGQGTQVTVSS |
| | Nucleotide sequence of heavy chain variable region | 15 | CAGGTGCAGCTGCAGGAAAGCGGCGGC GGCCTGGTGCAGGCGGGCGGCAGCCTGC GCCTGAGCTGCGCGGCGAGCGGCAATAT TTCTGCTGTTTACACTATGGGCTGGTATCG CCAGGCGCCGGGCAAAGAACGCGAATTT GTTGCCGCTATTGATTACGGTGGTAGTAC CTATTATGCGGATAGCGTGAAAGGCCGCT TTACCATTAGCCGCGATAACGCGAAAAAC ACCGTGTATCTGCAGATGAACAGCCTGA AACCGGAAGATACCGCGGTGTATTATTGC GCGTCTTACTACCATGGTTATTGGGGCCA GGGCACCCAGGTGACCGTGAGCAGC |
| NY-ESO-1-VHH-9 | CDR1 amino acid sequence | 16 | GYISYLNTM |
| | CDR2 amino acid sequence | 17 | EFVATIGQGSSTYY |
| | CDR3 amino acid sequence | 18 | AVYYCADQLGY |
| | Amino acid sequence of heavy chain variable region | 19 | QVQLQESGGGLVQAGGSLRLSCAASGYIS YLNTMGWYRQAPGKEREFVATIGQGSSTY YADSVKGRFTISRDNAKNTVYLQMNSLKP EDTAVYYCADQLGYWGQGTQVTVSS |
| | Nucleotide sequence of heavy chain variable region | 20 | CAGGTGCAGCTGCAGGAAAGCGGCGGC GGCCTGGTGCAGGCGGGCGGCAGCCTGC GCCTGAGCTGCGCGGCGAGCGGCTATATT TCTTACCTGAACACTATGGGCTGGTATCG CCAGGCGCCGGGCAAAGAACGCGAATTT GTTGCCACTATTGGTCAGGGTAGTAGTAC CTATTATGCGGATAGCGTGAAAGGCCGCT TTACCATTAGCCGCGATAACGCGAAAAAC ACCGTGTATCTGCAGATGAACAGCCTGA AACCGGAAGATACCGCGGTGTATTATTGC GCGGACCAGCTTGGTTATTGGGGCCAGG GCACCCAGGTGACCGTGAGCAGC |

### 3.2 Detection of Antigen Binding Specificity of Yeast Monoclones

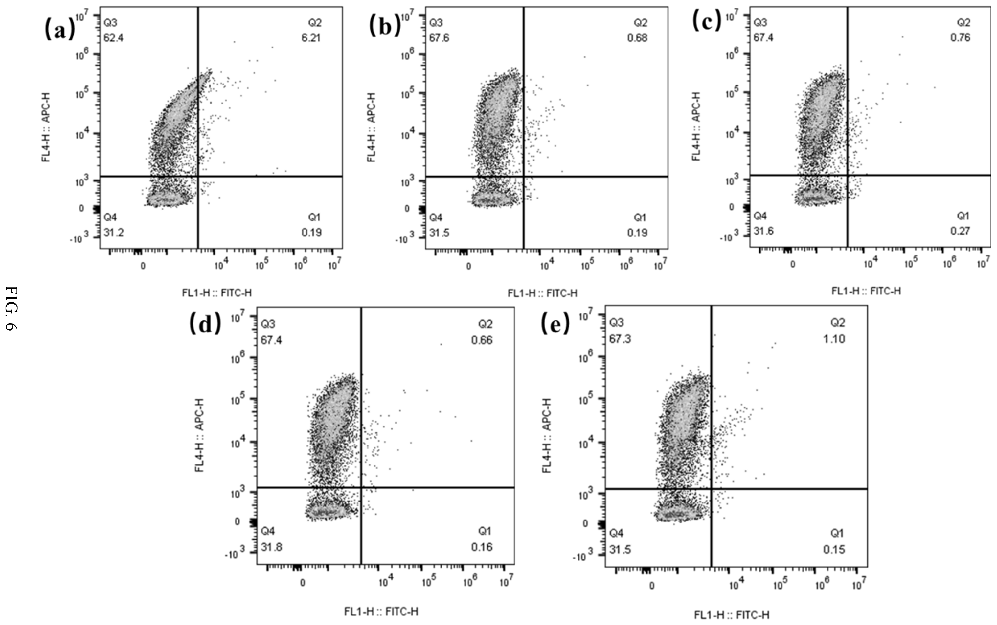

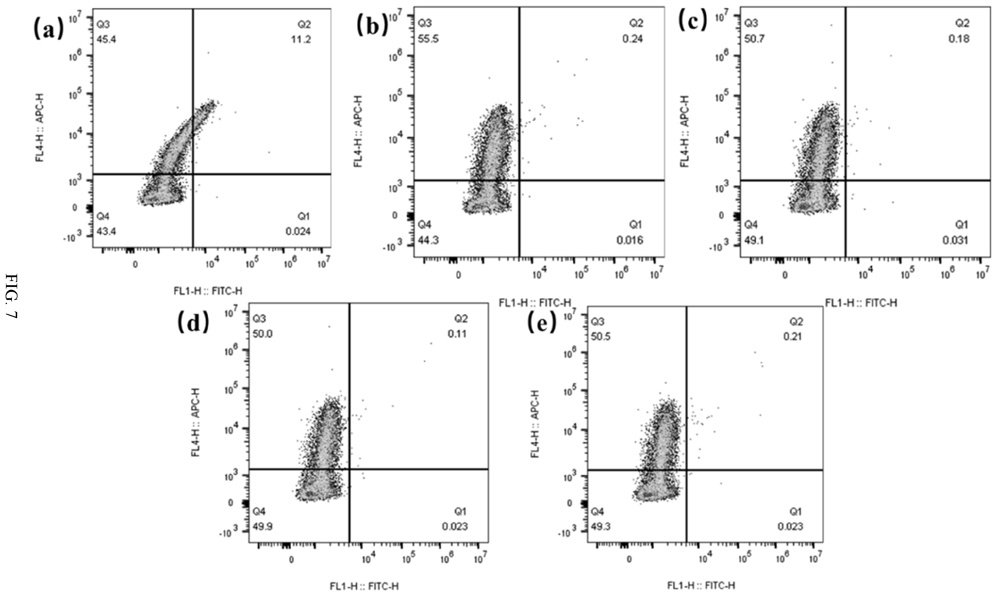

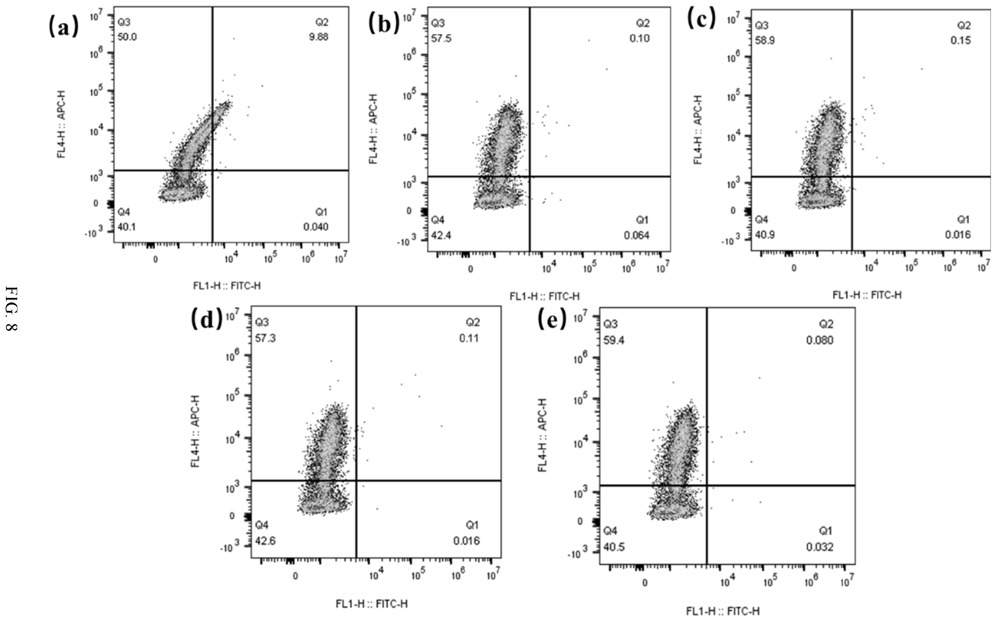

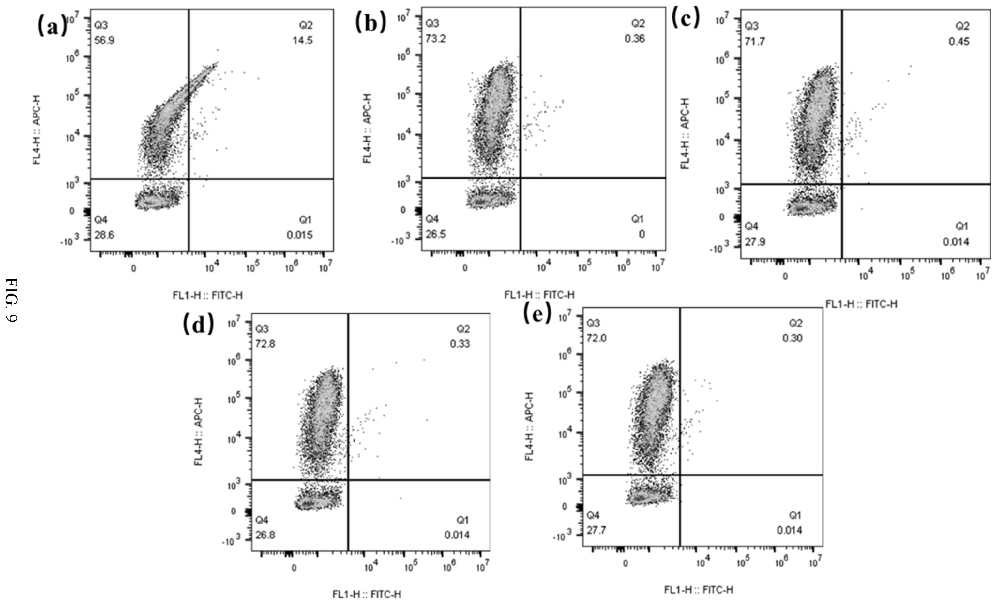

Each of the four yeast monoclonals was inoculated into 1 mL growth medium and cultured at 30 °C with shaking at 180 rpm for 24 h. The cultures were centrifuged at 2000 × g for 5 min at 4 °C, and the supernatants were discarded. The cell pellets were resuspended in 2 mL induction medium, and induced at 25°C with shaking at 180 rpm for 24 h. Detection was performed by flow cytometry, using APC-labeled anti-HA antibody and FITC-labeled Streptavidin protein for staining. The results are shown in FIG. 6 to FIG. 9. VHH-1, VHH-4, VHH-5 and VHH-9 monoclonals all showed strong binding to the positive antigen NY-ESO-1, but almost no binding to other negative antigens, indicating that the four screened monoclonals had strong binding specificity for NY-ESO-1 antigen.

### Example 4 Determination of Nanobody Affinity by Surface Plasmon Resonance (SPR)

Carboxyl groups on the chip surface were activated with 0.4 M EDC and 0.1 M NHS.

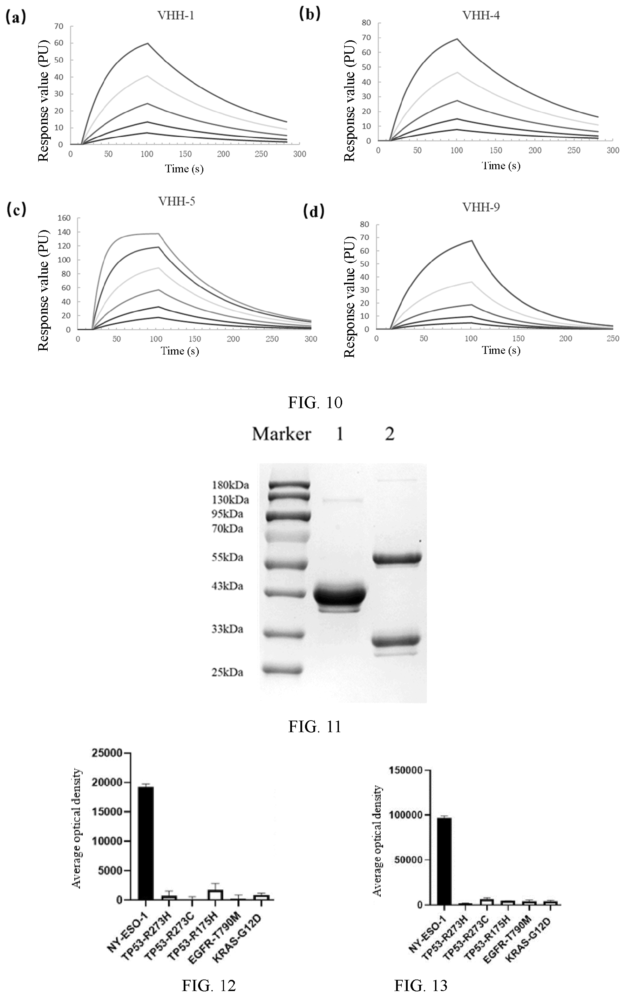

After activation, streptavidin was coupled onto the chip, and the remaining active sites were blocked with 1 M ethanolamine. The SLLMWITQC-HLA A0201 complex solution was flowed over the activated CM5 chip, so that the ligand was immobilized on the chip surface. Nanobody samples with serially diluted concentrations were injected sequentially, and changes in response signals were monitored. The BIAcore kinetic profiles of the binding of VHH-1, VHH-4, VHH-5, VHH-9 to the SLLMWITQC-HLA A0201 complex are presented in FIG. 10. The affinity data are shown in Table 3.

**Table 3 Affinity Data of VHH-1, VHH-4, VHH-5 and VHH-9**

| Name | Affinity Value |
|---|---|
| VHH-1 | 96.6nM |
| VHH-4 | 102nM |
| VHH-5 | 68.1nM |
| VHH-9 | 1170nM |

### Example 5 Specificity Verification of VHH-5 Nanobody

### a) Expression of Dimeric and Tetrameric Proteins

### Dimer expression plasmid VHH-GGGGS-Fc and tetramer expression plasmids

VHH-(GGGGS)₃-CH1-Fc and VHH-(GGGGS)₃-CL were constructed. The constructed plasmids were separately transfected into HEK293F cells. After 3 to 4 days, the cell cultures were centrifuged at 4000 × g for 20 min, and the cell supernatants were collected, filtered through 0.22 µm microporous membranes, and purified using an AKTA protein purification system equipped with a HiTrap Protein A affinity column. The purification steps were as follows: a. Turn on the AKTA protein purification system and the connected control computer. After the instrument is connected to the computer, set the pressure parameter (high pressure 0.25 MPa); b. Place the A and B pump heads in pure water filtered through a 0.45 µm membrane, set the flow rate (3 mL/min) and the flushing ratio of A and B pumps (50% B). After the pure water flushing reaches conductivity equilibrium (approximately 60 mL), connect the HiTrap Protein A affinity column (5 mL) to the AKTA purification system and continue flushing with pure water for at least three column volumes; c. Change the flushing ratio of A and B pumps to 0% B, and switch the A pump to loading buffer (50 mM Tris-HCl, pH 7.4). After conductivity equilibrium, switch the A pump to the culture supernatant to be purified. After completion of loading, switch the A pump back to loading buffer until conductivity equilibrium. Then adjust the proportion of B Pump to 100%, and switch the B pump to elution buffer (1 mol/L sodium acetate, pH 3.0) to elute the target protein. The AKTA purification system was flushed with pure water until conductivity equilibrium and the entire system was stored in 20% (v/v) ethanol. The tetramer and dimer proteins were ultrafiltered using ultrafiltration membranes with a molecular weight cutoff of 50 kDa, and the solvent was exchanged to PBS buffer. The protein concentration was determined using a Nanodrop ND-1000, and the protein samples were aliquoted and stored at -80°C for later use. The protein purity was analyzed by SDS-PAGE, and the results are shown in FIG. 11. The dimer and tetramer protein bands matched the expected molecular weights and had good purity.

### b) Specific Affinity Assay of VHH-5 Nanobody

T2 cells in the logarithmic growth phase were collected and centrifuged at 1000 rpm for 5 min. The supernatant was discarded, and cell pellets were washed once with PBS and resuspended in serum-free IMDM medium. The cells were seeded into 12-well plates at a density of 5 × 10⁵ cells per well, with a volume of 1 mL per well. Peptide was added to each well to a final concentration of 25 µg/mL. After thorough mixing, the plates were incubated for 2 h in a constant-temperature incubator at 37 °C with 5% CO₂ and saturated humidity. After incubation, cells were harvested by centrifugation at 500 × g for 5 min and washed once with PBS. A total of 200 µL dimeric or tetrameric protein solution (100 nM) was added to each well, and the mixture was incubated at 4 °C for 30 min. After washing twice with PBS, 200 µL goat anti-human IgG antibody (Beyotime, A0556) diluted at a ratio of 1:500 was added, and the mixture was incubated at 4°C for 30 min. After washing twice with PBS, 300 µL of PBS was added to resuspend the cells, and the FITC intensity was detected using an ACEA NovoCyte^{™} flow cytometer. The results are shown in FIG. 12 and FIG. 13. The dimer and tetramer proteins showed high binding intensity to the positive antigen NY-ESO-1, but almost no binding to other negative antigens, indicating that the VHH-5 nanobody had strong binding specificity for NY-ESO-1.

## Claims

1. An anti-NY-ESO-1 nanobody, wherein a VHH chain of the nanobody comprises three complementarity determining regions CDR1, CDR2, and CDR3, wherein an amino acid sequence of the CDR1 is any one of the sequences set forth in SEQ ID NO.11, SEQ ID NO.1, SEQ ID NO.6, and SEQ ID NO.16;
an amino acid sequence of the CDR2 is any one of the sequences set forth in SEQ ID NO.12, SEQ ID NO.2, SEQ ID NO.7, and SEQ ID NO.17; and
an amino acid sequence of the CDR3 is any one of the sequences set forth in SEQ ID NO.13, SEQ ID NO.3, SEQ ID NO.8, and SEQ ID NO.18.

2. The nanobody according to claim 1, wherein the VHH chain of the nanobody comprises three complementarity determining regions CDR1, CDR2, and CDR3 selected from any one of the following:
(1) a CDR1 having an amino acid sequence set forth in SEQ ID NO.11, a CDR2 having an amino acid sequence set forth in SEQ ID NO.12, and a CDR3 having an amino acid sequence set forth in SEQ ID NO.13;
(2) a CDR1 having an amino acid sequence set forth in SEQ ID NO.1, a CDR2 having an amino acid sequence set forth in SEQ ID NO.2, and a CDR3 having an amino acid sequence set forth in SEQ ID NO.3;
(3) a CDR1 having an amino acid sequence set forth in SEQ ID NO.6, a CDR2 having an amino acid sequence set forth in SEQ ID NO.7, and a CDR3 having an amino acid sequence set forth in SEQ ID NO.8;
(4) a CDR1 having an amino acid sequence set forth in SEQ ID NO.16, a CDR2 having an amino acid sequence set forth in SEQ ID NO.17, and a CDR3 having an amino acid sequence set forth in SEQ ID NO.18.

3. The nanobody according to claim 1, wherein an amino acid sequence of the VHH chain is any one of the sequences set forth in SEQ ID NO.14, SEQ ID NO.4, SEQ ID NO.9, and SEQ ID NO.19.

4. A nucleic acid molecule encoding the nanobody according to any one of claims 1 to 3.

5. The nucleic acid molecule according to claim 4, comprising a nucleotide sequence set forth in any one of SEQ ID NO.15, SEQ ID NO.5, SEQ ID NO.10, and SEQ ID NO.20.

6. An expression vector, comprising the nucleic acid molecule according to claim 4.

7. A host cell, wherein the host cell comprises the expression vector according to claim 6, or the nucleic acid molecule according to claim 4 is integrated into a genome of the host cell.

8. An immunoconjugate, comprising (a) the nanobody according to any one of claims 1 to 3; and (b) at least one conjugation moiety selected from the group consisting of a detectable marker, a drug, toxin, a cytokine, a therapeutic agent, a PK modification moiety, and an enzyme.

9. A pharmaceutical composition, comprising the nanobody according to any one of claims 1 to 3, the nucleic acid molecule according to claim 4 or the immunoconjugate according to claim 8, and a pharmaceutically acceptable carrier.

10. Use of the nanobody according to any one of claims 1 to 3, the nucleic acid molecule according to claim 4, the host cell according to claim 7, or the immunoconjugate according to claim 8, comprising any one of the following:
(1) use in preparation of a product for detecting NY-ESO-1;
(2) use in preparation of a product that binds to NY-ESO-1;
(3) use in preparation of a product for diagnosing or treating a tumor expressing NY-ESO-1.
